# EUROPÄISCHE PATENTSCHRIFT

(11) **EP 2 676 634 B1**
(45) Veröffentlichungstag und Bekanntmachung des Hinweises auf die Patenterteilung: **10.08.2016**
(21) Anmeldenummer: 13003000.0
(22) Anmeldetag: 12.06.2013
(51) Int. Cl.: A61F 2/00, D04B 1/00

(54) **Bandartige Struktur zur Augmentation eines Ligaments**
Tape-like structure for the augmentation a ligament
Structure de type bande pour l'augmentation d'un ligament

(30) Priorität: 22.06.2012 DE 202012006023 U
(43) Veröffentlichungstag der Anmeldung: 25.12.2013
(73) Patentinhaber: FEG Textiltechnik Forschungs- und Entwicklungsgesellschaft mbH, 52070 Aachen (DE)
(72) Erfinder: Jäger, Wolfram, 40477 Düsseldorf (DE); Obolenski, Boris, 52070 Aachen (DE); Müllen, Andreas, 52072 Aachen (DE)
(74) Vertreter: Gottschald, Jan

(56) Entgegenhaltungen:
- EP-A1- 1 600 118
- WO-A1-2011/115340
- GB-A- 2 474 866
- US-A1- 2006 229 493
- US-A1- 2009 319 053

## Beschreibung

Die Erfindung betrifft eine bandartige Struktur zur Augmentation eines Ligaments mit den Merkmalen des Oberbegriffs von Anspruch 1 sowie ein Verfahren zur Herstellung einer solchen bandartigen Struktur mit den Merkmalen des Anspruchs 12.

Die hier gegenständliche bandartige Struktur dient als medizinisches Implantat für die Verstärkung bzw. den Ersatz von Bändern im Beckenbereich. Diese Bänder im kleinen Becken werden mit zunehmendem Alter häufig schwächer oder versagen vollständig, was etwa bei weiblichen Patienten zu einer Gebärmutteroder Scheidensenkung führen kann. Ab einer bestimmten Schwere spricht man sogar von einem entsprechenden Prolaps. Diese Verschiebung des Uterus oder der Vagina führt häufig auch zu einer Verschiebung der Lage der Hamblase im Subperitonealraum oder zu einer Abweichung der auf die Harnblase einwirkenden Kräfte im Vergleich zum gesunden Zustand. Inkontinenz ist dann häufig die Folge.

Aus dem Stand der Technik ist es bekannt, solche Verschiebungen oder Prolapse durch eine künstliche Aufhängung der betroffenen Organe, welche vormals durch die körpereigenen Bänder gehalten wurden, an anatomisch geeigneten Befestigungspunkten zu behandeln.

So zeigt die US 6,575,897 eine Aufhängevorrichtung mit einem flächigen Mitteltextil und zwei ebenfalls flächigen Seitentextilen aus dem gleichen textilen Material, die miteinander durch ein schnurartiges Band, welches ebenfalls aus dem identischen textilen Material besteht, verbunden sind. Das Band ist an das Mitteltextil - welches auch als Mittelabschnitt bezeichnet werden kann - und an die beiden Seitentextile - entsprechend als Seitenabschnitte anzusehen - angenäht oder angeschweißt. Während eines operativen Eingriffs wird das Mitteltextil nun an dem zu stützenden Organ befestigt. Diese Stelle kann auch als Zugpunkt bezeichnet werden. Regelmäßig handelt es sich dabei etwa entweder um die Gebärmutter oder für den häufigen Fall, dass eine Hysterektomie bereits vorgenommen wurde, um den verbliebenen Zervix oder den Scheidenstumpf. Die beiden Seitcntextile werden dann durch Öffnungen in dem Peritoneum aus dem Subperitonealraum geführt, um anschließend an einer geeigneten, häufig knöchernen Befestigungsstelle wie etwa dem Kreuzbein befestigt zu werden. Auf diese Weise wird das betroffene Organ wieder in seine ursprüngliche Lage aufgerichtet und eine etwaige Verschiebung wieder beseitigt.

Jedoch weist diese Aufhängevorrichtung zur Augmentation der Bänder einige bedeutende Nachteile auf. Damit ein langfristig belastbarer Kontakt sowohl an dem Zugpunkt entsprechend der zu stützenden Stelle - beispielsweise dem Zervix - als auch an den Befestigungspunkten - hier das Kreuzbein - entstehen kann, sind möglichst große Maschen im Textil von Vorteil, da diese das Hineinwachsen des Gewebes in die Maschen des Textils begünstigen. Speziell an den Abschnitten, an denen gemäß dem Stand der Technik das Band auf dem Mitteltextil oder den Seitentextilen angebracht ist, ist aber die wirksame Porosität der Struktur durch das Übereinanderliegen der beiden Textilien sehr deutlich vermindert. Offenkundig ist, dass in der Praxis keine identische Abdeckung der Maschen des Bandes mit den Maschen des Mitteltextils oder der Seitentextile erreicht werden kann, weswegen die dann an diesen Stellen entstehende Porosität im Prinzip schon aus diesem Grund nur noch halb so groß ist gegenüber dem Zustand ohne Übereinanderliegen.

Zu beachten ist hierbei, dass es für die Porosität, welche grundsätzlich als prozentualer Anteil der Öffnungsfläche an der Gesamtfläche des Textils definiert ist, nicht auf die Porosität des betroffenen Textils in seinem Ursprungszustand ankommt, sondern vielmehr auf die effektive Porosität nach seiner Anwendung. Die Porosität im Ausgangszustand wird etwa dadurch verringert, dass durch die Fremdkörperreaktion des Körpers unweigerlich Granulome und Narbengewebe die einzelnen Stränge des Textils umschließen, wodurch natürlich Öffnungsfläche verloren geht. Im Extremfall kann durch diese Reaktion ein vollständiger Verschluss der die Poren bildenden Netzmaschen erfolgen. Der hier maßgebliche Begriff der effektiven Porosität, welcher diese Beeinträchtigungen berücksichtigt, ist dem Fachmann aus dem Aufsatz "New Objective Measurement to Characterize the Porosity of Textile Implants" von Mühl, Binnebösel, Klinge und Goedderz bekannt (Journal of Biomedical Materials Research Part B: Applied Biomaterials, 2007.84B(1): p. 176-183), auf welchen hiermit ausdrücklich Bezug genommen wird.

Das Aufeinanderliegen der beiden textilen Netzstrukturen des Mitteltextils bzw. des Seitentextils und des Bandes führt also zu einer überproportionalen Verminderung der effektiven Porosität im genannten Sinne, wodurch das Einwachsen des Implantats in das Gewebe speziell am Zugpunkt deutlich erschwert wird.

Dieses Problem wird zusätzlich dadurch verschärft, dass das Übereinanderliegen der Textilien nicht nur die Porosität, sondern an diesen Abschnitten auch die Biegsamkeit des Materials insgesamt beeinträchtigt. Dadurch ist ein genaues Anschmiegen an das Gewebe nicht mehr so leicht möglich, was aber für das bereits erwähnte Hineinwachsen des Gewebes in das Textil ebenfalls von Vorteil wäre.

Aus diesen beiden Gründen ist also gerade an den besonders wichtigen Stellen, an denen der eigentliche Kontakt zu dem Zugpunkt und zu den Befestigungspunkten hergestellt wird, ein schlechtere Halt gegeben als an den Randbereichen des Mitteltextils oder der Seitentextile.

Hinzu kommt, dass über die gesamte Einsatzdauer der Aufhängevorrichtung die Befestigung des Bandes an dem Mitteltextil und an beiden Seitentextilen unbedingt halten muss. Bei einer Loslösung der Schweiß- oder Nähverbindung - aus welchem Grund auch immer - schon an nur einer dieser Stellen kann das Organ nicht mehr gestützt werden und es tritt im Prinzip dieselbe Situation ein wie vor dem Einsatz der Aufhängevorrichtung, mit der entsprechenden Notwendigkeit eines weiteren Eingriffs beim Patienten.

Ferner weist das bekannte textile Material keine Vorkehrungen gegen ein Einschnüren auf. Die Aufhängevorrichtung ist nicht nur einer konstanten, sondern auch einer dynamischen Zugbelastung ausgesetzt, welche sich etwa durch den unterschiedlichen Füllgrad der Harnblase und die Körperbewegungen des Patienten insgesamt ergibt. Diese Zugbelastung der Aufhängevorrichtung kann aber zu einer einschnürenden Verformung der einzelnen Maschen des textilen Materials führen. Durch eine solche Einschnürung werden nicht nur die Maschen in ihrer Fläche verkleinert, was zu einer entsprechenden Verminderung der effektiven Porosität führt, sondern es werden auch bereits entstandene Verwachsungen zwischen Gewebe und den einzelnen Maschen wieder getrennt und aufgerissen. Auch dies verlangsamt den Einwachsprozess und mindert die Qualität der Befestigung der Aufhängevorrichtung.

Aus der EP 1 600 118 A1 bekannt ist ein Implantat zur Suspension der Harnblase bei Senkungsbeschwerden und/oder bei Harninkontinenz der Frau mit einer etwa rechteckigen, ovalen oder langgestreckt trapezförmigen Basis, von deren Längsseiten in einem Abstand voneinander bandförmige Fortsätze abstehen, wobei die bandförmigen Fortsätze die gleiche Bindungsart aufweisen wie die Basis und mit dieser aus einem Stück bestehen. Allerdings weist dieses Implantat keinen Abschnitt zur Aufnahme einer Verzwirbelung der Fortsätze auf.

Folglich besteht das der Erfindung zugrundeliegende Problem darin, die aus dem Stand der Technik bekannte bandartige Struktur zur Augmentation eines Ligaments und ihr entsprechendes Herstellungsverfahren derart auszugestalten und weiterzubilden, dass eine bessere Verbindung zwischen ihr und dem Gewebe an den Kontaktstellen erreicht wird.

Das obige Problem wird bei einer bandartigen Struktur zur Augmentation eines Ligaments gemäß dem Oberbegriff von Anspruch 1 durch die Merkmale des kennzeichnenden Teils von Anspruch 1 gelöst.

Bezogen auf das Herstellungsverfahren wird das obige Problem durch den Gegenstand des Anspruchs 12 gelöst.

Wesentlich ist die Erkenntnis, dass eine bessere Verbindung zwischen der zu implantierenden bandartigen Struktur und der aufzuhängenden Gewebestelle dadurch erreicht werden kann, dass der bandartige Teil, welcher die Verbindung zwischen der aufzuhängenden Stelle - dem Zugpunkt - und den Befestigungspunkten herstellen soll, und der Mittelabschnitt zur Verbindung mit eben diesem Zugpunkt aus ein und demselben gewirkten Textil bestehen. Dieser bandartige Teil umfasst eine Mehrzahl von Fäden. Die Irregularität in der textilen Struktur, die aus der nachträglichen Befestigung des bandartigen Textils am eigentlichen flächigen Kontaktteil gemäß dem Stand der Technik rührt und die mit ihr verbundenen, soeben beschriebenen Nachteile können dann vollständig vermieden werden.

Auf diese Weise kann der Mittelabschnitt als ein für die Kontaktierung mit dem Gewebe optimiertes Gewirke ausgelegt werden und wird darin nicht durch andere überlagerte Strukturen beeinträchtigt. Unter dem erfindungsgemäßen Gewirke ist ein Kettengewirke in Häkelgalontechnik zu verstehen. Ein solches Gewirke kann durch eine Häkelgalonmaschine hergestellt werden, also eine Kettenwirkmaschine mit Schusseintragungssystem.

Mittelkettrichtung im Sinne der Erfindung bedeutet die Kettrichtung des Mittelgewirkes. Diese Kettrichtung entspricht der Herstellungsrichtung des Textils in der Häkelgalonmaschine, also derjenigen Richtung, in welche sich die Wirknadeln bei der Anordnung der Kettfäden des Kettengewirks weiterbewegen. Orthogonal zu der Kettrichtung in der Ebene des jeweiligen Gewirkes liegt die Schussrichtung, welche der grundsätzlichen Ausrichtung der Schussfäden entspricht. Dabei sind beide denkbaren orthogonalen Richtungen zur Kettrichtung in der Ebene des Gewirkes als Schussrichtung zu verstehen.

Das Umfassen der Mehrzahl von Fäden durch das Mittelgewirke ist so zu verstehen, dass diese Fäden in irgendeiner Form integraler Bestandteil des Kettengewirkes sind. Dies ist genau dann der Fall, wenn jeder einzelne Faden der Mehrzahl von Fäden entweder als Kettfaden oder als Schussfaden bei der Herstellung des Kettengewirkes verwendet wurde, wobei auch einige Fäden als Schuss- und andere als Kettfaden verwendet worden sein konnten. Jeder einzelne dieser Fäden kann aus einem Multifil oder einem Monofilament bestehen.

Als bandartige Struktur zur Verbindung zwischen Mittel- und Seitenabschnitten kann dann entsprechend eine Verlängerung von einigen der für die Herstellung dieses Gewirkes verwendeten Fäden eingesetzt werden. Die eigentliche Kontaktstelle bleibt also von dieser Maßnahrne - der auch anderweitigen Verwendung der benutzten Fäden - vollkommen unberührt.

Wie bereits beschrieben wurde, ist die Aufhängevorrichtung regelmäßig einer Zugbelastung ausgesetzt Mittels der Erfindung lässt sich gewährleisten, dass eine solche Zugbeanspruchung von den genannten Fäden aufgenommen wird und auch durch das den Mittelabschnitt bildende Gewirke hindurch mittels eben diese Fäden selbst weitergeleitet werden kann, ohne dass eine wesentliche Verformung des Mittelabschnitts eintritt. Vor allem tritt keine Einschnürung der Maschen des Mittelabschnitts auf, sodass die hohe effektive Porosität des Mittelabschnitts gewahrt werden kann.

Die Ausbildung als Gewirke ermöglicht es ferner, maschinell diese komplexe Struktur mit einer Häkelgalonmaschine gleichermaßen aus einem Guss herzustellen.

Die bevorzugten Ausgestaltungen gemäß den Ansprüchen 2 und 3 beschreiben Ausgestaltungen eines Verbindungsabschnitts zwischen dem Mittelabschnitt - welcher den Zugpunkt kontaktieren soll - und den Seitenabschnitt zur Kontaktierung an den Befestigungspunkten. Dieser Verbindungsabschnitt soll gerade keine Verbindung mit Gewebe eingehen, sondern vielmehr möglichst kontaktfrei die Entfernung zwischen dem Zugpunkt und den Befestigungspunkten überbrücken. Die bevorzugten Ausgestaltungen sehen vor, dass der Verbindungsabschnitt zumindest wesentlich aus den Fäden besteht, welche den Mittelabschnitt und den Seitenabschnitt verbinden. Damit kann erreicht werden, dass Zugkräfte auf die erfindungsgemäße bandartige Struktur von einem Seitenabschnitt bis zum Mittelabschnitt und ggf. zu weiteren vorhandenen Seitenabschnitten vollständig von den genannten Fäden übertragen werden. Auf diese Weise können speziell diese Fäden zur Aufnahme der Zugkräfte ausgelegt werden. Ferner werden Verformungs- und Einschnüreffekte bei den Maschen der bandartigen Struktur vermieden.

Da die bandartige Struktur zusammengerollt durch ein Loch in den Subperitonealraum geführt wird, ist es sehr gut möglich, dass beim anschließenden Ausbreiten des Mittelabschnitts und der Seitenabschnitte diese - im Hinblick auf ihre Verbindung - zueinander verdreht angeordnet sind. Deswegen ist vorschlagsgemäß bei der bandartigen Struktur ein spezieller Verzwirbelungsabschnitt vorgesehen, welcher eine solche Verdrehung lokal aufnehmen kann, ohne dass dadurch Kräfte auf den Mittelabschnitt oder die Seitenabschnitte ausgeübt werden, welche diese von ihrer jeweiligen Kontaktfläche lösen könnten.

Die bevorzugte Ausgestaltung des Anspruchs 5 sieht vor, dass auch die Seitenabschnitte je ein Kettengewirke aufweisen, welches die Mehrzahl von Fäden im selben Sinne umfasst wie der Mittelabschnitt. Die erfindungsgemäß für den Mittelabschnitt beschriebenen Vorteile treten damit auch für die Seitenabschnitte ein.

Anspruch 6 beschreibt eine bevorzugte Ausgestaltung, welche eine ad-hoc Längenanpassung der bandartigen Struktur besonders vereinfacht.

Durch die bevorzugte Längsorientierung der rechteckigen Maschenöffnungen des Mittelabschnitts in Kettrichtung gemäß der bevorzugten Ausgestaltung nach Anspruch 8 wird die Porosität vergrößert - indem beispielsweise auf die Hälfte der Querstege bei quadratischen Maschenöffnungen verzichtet wird - ohne das Ansprechen auf Zugbelastung der bandartigen Struktur zu beeinträchtigen.

Der Gegenstand des Anspruchs 10 wiederum optimiert das Einführen der bandartigen Struktur in den Subperitonealraum.

Die besonders bevorzugte Ausführungsform des Anspruchs 11. wiederum ermöglicht es, postoperativ den weiteren Behandlungserfolg der implantierten bandartigen Struktur zu überwachen.

Im Folgenden wird die Erfindung anhand einer lediglich ein Ausführungsbeispiel darstellenden Zeichnung näher erläutert. In der Zeichnung zeigt

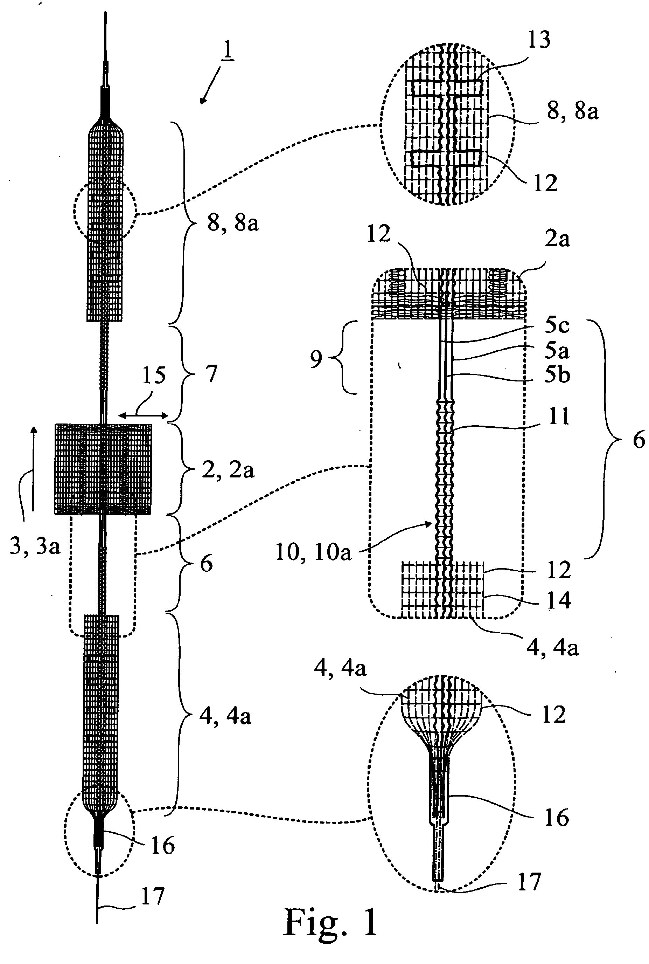

Fig. 1 eine vorschlagsgemäße bandartige Struktur zur Augmentation eines Ligaments vor dem Einsetzen in einer Draufsicht.

Die in der Zeichnung dargestellte erfindungsgemäße bandartige Struktur 1 zur Augmentation eines Ligaments ist zur Behandlung einer Absenkung oder eines Prolapses eines Organes im kleinen Becken geeignet, insbesondere des Uterus oder der Vagina. Hierbei werden die Bänder, welche das betreffende Organ im gesunden Zustand festhalten sollen, durch die bandartige Struktur 1 unterstützt oder ersetzt.

Wie in der Fig. 1 dargestellt ist, umfasst die erfindungsgemäße bandartige Struktur 1 einen Mittelabschnitt 2 zur Befestigung an einem Organ des kleinen Beckens. Die Befestigung an dem Organ kann durch ein Kammern, ein Nähen oder auf eine andere Weise erfolgen. Mittelfristig soll der Mittelabschnitt 2 in das Gewebe einwachsen. Die Stelle des Organs, an welcher der Mittelabschnitt 2 befestigt wird, stellt den Zugpunkt dar in dem Sinne, dass diese Stelle durch die bandartige Struktur 1 dadurch gestützt werden soll, dass auf diese Stelle eine Zugkraft ausgeübt wird.

Der Mittelabschnitt 2 weist ein Mittelgewirke 2a - welches ein Kettengewirke ist - mit einer Mittelkettrichtung 3 auf. Die Mittelkettrichtung 3 entspricht einfach der Kettrichtung des Kettengewirkes, welches das Mittelgewirke 2a ist.

Die erfindungsgemäße bandartige Struktur 1 umfasst ferner einen Seitenabschnitt 4 zur Befestigung an einer Aufhängungsfläche. Diese Aufhängungsfläche stellt denjenigen festen Befestigungspunkt dar, welcher über die bandartige Struktur 1 den Zugpunkt stützt. Regelmäßig handelt es sich bei dem Befestigungspunkt um eine knöcherne oder knochenbezogene Struktur wie etwa eine Stelle am Kreuzoder Steißbein. Neben einer Befestigung am Knochen selbst kommt ebenso eine Befestigung an der den Knochen umgebenden Knochenhaut in Frage. Darüber hinaus kann der Befestigungspunkt auch an einem Ligament gewählt werden.

Erfindungsgemäß umfasst die bandartige Struktur 1 weiter eine Mehrzahl von Fäden 5a, 5b, 5c, welche mit dem Mittelabschnitt 2 und dem Seitenabschnitt 4 verbunden sind. Im hier dargestellten Fall handelt es sich um drei Fäden 5a, 5b, 5c.

Grundsätzlich sind dabei erfindungsgemäß jedwede weitere Einzelheiten zu Anordnung oder Verhältnis untereinander der Mehrzahl von Fäden 5a, 5b, 5c frei wählbar. Diese können parallel und in einer Linie oder jeweils für sich mäandemd verlaufen. Sie können untereinander ohne jede Verbindung sein, untereinander verflochten oder mittels weiterer Fasern oder Fäden zumindest streckenweise verknüpft sein.

Erfindungsgemäß ist nun weiter vorgesehen, dass das Mittelgewirke 2a die Mehrzahl von Fäden 5a, 5b, 5c umfasst. Jeder einzelne Faden 5a, 5b, 5c ist somit entweder als Kett- oder als Schussfaden in dem Mittelgewirke 2a eingearbeitet. Auf diese Weise gibt es keine Kontaktierungssstellen im eigentlichen Sinne zwischen der Mehrzahl von Fäden 5a, 5b, 5c und dem Mittelabschnitt 2. Eine Zugkraft auf die Mehrzahl von Fäden 5a, 5b, 5c wirkt also an genau definierten Stellen auf den Mittelabschnitt 2.

Bevorzugt ist hierbei, dass jeder einzelne Faden der Mehrzahl von Fäden 5a, 5b, 5c als Geflecht von Monofilamenten ausgebildet ist.

In einer bevorzugten Ausführungsform weist die bandartige Struktur 1 einen Verbindungsabschnitt 6 auf, welcher die Mehrzahl von Fäden 5a, 5b, 5c umfasst. Dabei ist der Verbindungsabschnitt 6 zwischen dem Mittelabschnitt 2 und dem Seitenabschnitt 4 so angeordnet, dass eine auf den Mittelabschnitt 2 und den Seitenabschnitt 4 ausgeübte Zugkraft zumindest abschnittsweise vollständig von dem Verbindungsabschnitt 6 übertragen wird. Mit anderen Worten ausgedrückt bedeutet dies, dass wenn eine Zugkraft auf den Mittelabschnitt 2 und den Seitenabschnitt 4 ausgeübt wird, der Übertragungsweg dieser Zugkraft von dem Mittelabschnitt 2 zum Seitenabschnitt 4 (oder umgekehrt) zumindest eine Teilstrecke umfasst, auf welche die Zugkraft ausschließlich von dem Verbindungsabschnitt 6 übertragen wird. Es gibt also keinen parallel verlaufenden weiteren Zweig auf dieser Strecke, welcher daneben diese Zugkraft übertragen könnte. Besonders bevorzugt ist dabei, dass die ausgeübte Zugkraft zumindest abschnittsweise vollständig von der Mehrzahl von Fäden 5a, 5b, 5c, übertragen wird, wobei die zumindest abschnittsweise vollständige Übertragung so wie eben beschrieben zu verstehen ist.

Hierbei kann der Verbindungsabschnitt 6 auch in einem einzigen Herstellungsdurchgang mittels einer Häkelgalonmaschine mit dem Mittelabschnitt 2 zusammen hergestellt worden sein.

Eine optimale und ausbalancierte Aufhängung wird erreicht, wenn die bandartige Struktur 1 an zwei Befestigungspunkten aufgehängt wird, wobei die zugehörigen Seitenabschnitte jeweils an gegenüberliegenden Seiten des Mittelabschnitts 2 angeordnet sind. Aus diesem Grunde ist in einer bevorzugten Ausführungsform vorgesehen, dass die bandartige Struktur 1 einen zweiten Verbindungsabschnitt 7 und einen zweiten Seitenabschnitt 8 aufweist, wobei je ein Verbindungsabschnitt 6, 7 zwischen dem Mittelabschnitt 2 und den Seitenabschnitten 4, 8 so angeordnet ist, dass eine auf die beiden Seitenabschnitte 4, 8 ausgeübte Zugkraft zumindest abschnittsweise vollständig von den Verbindungsabschnitten 6, 7 übertragen wird. Bevorzugt ist, dass die ausgeübte Zugkraft zumindest abschnittsweise vollständig von der Mehrzahl von Fäden 5a, 5b, 5c übertragen wird. Wiederum ist die zumindest abschnittsweise vollständige Übertragung so wie obenstehend beschrieben zu verstehen.

Jedwede den Verbindungsabschnitt 6 betreffende Feststellung gilt bevorzugt auch für den zweiten Verbindungsabschnitt 7. Analog kann jedes bevorzugte Merkmal des Seitenabschnitts 4 ebenso auch auf den zweiten Seitenabschnitt 8 bezogen werden.

Wie bereits beschrieben ist davon auszugehen, dass sich während des operativen Eingriffs zur Implantierung der bandartigen Struktur 1 der Mittelabschnitt 2 und ein Seitenabschnitt 4 gegeneinander um den Verbindungsabschnitt 6 verdrehen. Um diese Drehung kontrolliert aufzunehmen, ist bevorzugt vorgesehen, dass der Verbindungsabschnitt 6, 7 einen Verzwirbelungsabschnitt 9 aufweist, welcher aus der Mehrzahl von Fäden 5a, 5b, 5c besteht. Noch weiter bevorzugt ist, dass die Mehrzahl von Fäden 5a, 5b, 5c in dem Verzwirbelungsabschnitt 9 getrennt geführt ist. Die getrennte Führung als einzelne Fäden 5a, 5b, 5c erlaubt auch ohne weiteres eine mehrmalige Verzwirbelung der Fäden 5a, 5b, 5c, ohne dass auf die anderen Teile der bandartigen Struktur 1 dadurch eine Kraft ausgeübt würde.

Weiter ist bevorzugt, dass die Mehrzahl von Fäden 5a, 5b, 5c abschnittsweise ein Textilband 10 bildet, in welchem die Mehrzahl von Fäden 5a, 5b, 5c durch Bindefasern 11 miteinander verbunden ist. Insbesondere kann das Textilband aus der Mehrzahl von Fäden 5a, 5b, 5c und dem Textilband 10 bestehen.

Bei dem Textilband 10 kann es sich auch um ein Gewirke, insbesondere ein Kettengewirke handeln, welches die Mehrzahl von Fäden 5a, 5b, 5c umfasst. In diesem Fall stellen auch die Bindefasern 11 Kett- oder Schussfäden dieses Kettengewirkes dar. Bei diesem Kettengewirke kann es sich wiederum um eines handeln, was im selben Arbeitsgang wie das Mittelgewirke 2a hergestellt wurde. Wenn sie nicht durch einen nur aus den losen Fäden 5a, 5b, 5c bestehenden Verzwirbelungsabschnitt 9 voneinander getrennt werden, können dieses Gewirke und das Mittelgewirke 2a auch ein einzelnes zusammenhängendes Gewirke bilden.

Der bereits oben beschriebene, zur Aufnahme von Verdrehungen vorgesehene Verzwirbelungsabschnitt 9 kann grundsätzlich in seiner Länge relativ kurz im Vergleich zur gesamten Länge des Verbindungsabschnitts 6, 7 ausgeführt werden, da bereits eine solche kurze Länge durchaus dazu ausreicht, mehrere Verdrehungen aufzunehmen. Die gesamte Länge des Verbindungsabschnitts 6, 7 hingegen richtet sich nach der anatomischen Entfernung, welche zwischen dem Zugpunkt und den Befestigungspunkten zu überbrücken ist. Diese anatomische Entfernung ist regelmäßig größer. Um nun die auftretenden Verzwirbelungen auf einen bestimmten Bereich des Verbindungsabschnitts 6, 7 einzuschränken ist bevorzugt vorgesehen, dass der Verbindungsabschnitt 6, 7 einen Übergangsabschnitt 10a aufweist, welcher aus dem Textilband 10 besteht. Da der Übergangsabschnitt 10a Querverbindungen zwischen der Mehrzahl von Fäden 5a, 5b, 5c aufweist, kann hier nicht ohne weiteres eine Verzwirbelung auftreten. Diese bleiben damit auf den Verzwirbelungsabschnitt 9 beschränkt. Die Folge ist ein definiertes Verhalten des Verbindungsabschnitts 6, 7.

Um eine besonders gute Belastbarkeit der bandartigen Struktur 1 auf Zug zu erreichen, bildet gemäß einer vorteilhaften Ausführungsform die Mehrzahl von Fäden 5a, 5b, 5c Kettfäden 12 in dem Mittelgewirke 2a. Damit geht einher, dass die Mehrzahl von Fäden 5a, 5b, 5c in dem Mittelgewirke 2a entlang der Mittelkettrichtung 3 verläuft. Herstellungsbedingt ist ein Kettengewirk in Kettrichtung besonders gut auf Zug belastbar.

Um die bereits beschriebenen Vorteile, welche sich aus einer Ausbildung des Mittelabschnitts 2 als Mittelgewirke 2a ergeben, gleichermaßen für die Seitenabschnitte 4, 8 zu erhalten, ist gemäß einer bevorzugten Ausführungsform vorgesehen, dass der Seitenabschnitt 4, 8 ein Seitengewirke 4a, 8a aufweist, welches die Mehrzahl von Fäden 5a, 5b, 5c umfasst. Dabei ist das Umfassen der Mehrzahl von Fäden 5a, 5b, 5c durch das Seitengewirke 4a, 8a in dem gleichen Sinne zu verstehen wie das bereits beschriebene Umfassen der Mehrzahl von Fäden 5a, 5b, 5c durch das Mittelgewirke 2a. Bevorzugt besteht das Seitengewirke 4a, 8a aus der Mehrzahl von Fäden 5a, 5b, 5c. Bevorzugt ist auch, dass die Mehrzahl von Fäden 5a, 5b, 5c Kettfäden 12 in dem Seitengewirke 4a, 8a bildet, wodurch sich die oben genannten Vorteile hinsichtlich der Zugbelastung hier auch ergeben. Insbesondere vorteilhaft ist es, dass das Seitengewirke 4a, 8a die Bindefasern umfasst. Das bedeutet, dass die Bindefasern auch Kett- oder Schussfäden des Seitengewirkes 4a, 8a bilden.

Wiederum kann es sich bei den Seitengewirken 4a, 8a um solche handeln, die im selben Arbeitsgang wie das Mittelgewirke 2a hergestellt wurden. Auch für sie gilt, dass wenn sie nicht etwa durch einen nur aus den losen Fäden 5a, 5b, 5c bestehenden Verzwirbelungsabschnitt 9 von dem Mittelgewirke 2a getrennt werden, mit dem Mittelgewirke 2a und ggf. einem ein Gewirke bildenden Textilband 10 auch ein einzelnes zusammenhängendes Gewirke bilden können.

Der tatsächliche Abstand zwischen dem Zugpunkt und den Befestigungspunkten hängt nicht nur in erster Linie von dem zu stützenden Organ und in zweiter Linie von der speziellen Wahl des Zugpunktes und der Befestigungspunkte ab, sondern auch von der Größe und den anatomischen Dimensionen des jeweiligen Patienten. Um die bandartige Struktur 1 für eine große Bandbreite solcher Randbedingungen einsetzbar zu halten, ist regelmäßig der Seitenabschnitt 4, 8 in seiner Länge überdimensioniert und erlaubt somit einen ad hoc Zuschnitt unmittelbar vor oder sogar während des Eingriffs. Allerdings ist die tatsächliche Längenmessung an der bandartigen Struktur 1 schwierig, sobald sich die bandartige Struktur 1 erst im Subperitonealraum befindet. Um dem Operateur einen Anhaltspunkt für den Ansatz des Zuschnitts zu bieten ist bevorzugt vorgesehen, dass die Mehrzahl von Fäden 5a, 5b, 5c so angeordnet ist, dass sie Markierungen 13 zur Kennzeichnung von Schneide- oder Verbindungsstellen bildet. Dies kann etwa dadurch geschehen, dass die Mehrzahl von Fäden 5a, 5b, 5c von den anderen Bestandteilen des Seitenabschnitts 4, 8 farblich abgesetzt ist, wie auch in der Fig. 1 dargestellt ist. Die Markierungen 13 können dann jeweils durch einen unregelmäßigen Verlauf zumindest eines der Mehrzahl von Fäden 5a, 5b, 5c gebildet werden. Gemäß der in der Fig. 1 gezeigten Situation kann dies durch eine abschnittsweise in Schussrichtung versetzte Anordnung der zum Rand hin angeordneten Fäden 5a, 5c geschehen. Anstatt also eine direkte Messung der Länge vorzunehmen, kann der Operateur eine bestimmte Schneidstelle mit einer bestimmten Zahl von abzuzählenden Markierungen 13 identifizieren. Dabei sind die Markierungen 13 vorzugsweise zueinander regelmäßig beabstandet.

Diese Markierungen 13 dienen aber nicht nur der Bestimmung von Schneidstellen, sondern können auch diejenigen Stellen der bandartigen Struktur 1 kennzeichnen, an welchen genau die Befestigung vorgenommen werden soll. So kann etwa ein zur Befestigung mittels einer Naht vorgesehener Faden oder eine Klammer durch die bandartige Struktur 1 an der durch eine solche Markierung 13 identifizierten Stelle geführt werden.

Eine optimale Zugfestigkeit der bandartigen Struktur 1 mit der gleichzeitigen Möglichkeit einer sehr eleganten und zuverlässigen Herstellungsweise bietet sich dann, wenn die bandartige Struktur 1 eine gemeinsame Kettrichtung 3a aufweist. Diese entspricht dann der Mittelkettrichtung 3. Eine gemeinsame Kettrichtung 3a kann etwa dadurch erreicht werden, dass zumindest die textilen Teile der bandartigen Struktur 1 ein Kettengewirke bilden, das durch einen einzelnen Arbeitsgang einer Häkelgalonmaschine hergestellt wurde.

Bevorzugt ist in diesem Zusammenhang, dass die Mehrzahl von Fäden 5a, 5b, 5c Kettfäden entlang der bandartigen Struktur 1 bilden, also jedenfalls entlang des textilen Teils der bandartigen Struktur 1 Kettfäden bilden. Weiter ist bevorzugt, dass alle Kanten der bandartigen Struktur 1 entlang der gemeinsamen Kettrichtung 3a echte Kanten sind. Eine echte Kante ist eine solche, welche keine freistehenden Fadenenden aufweist. Indem freistehende Fadenenden vermieden werden, wird auch das durch sie begründete Verletzungsrisiko am umgebenden Gewebe vermindert.

Vorzugsweise weist der Mittelabschnitt 2 rechteckige Maschenöffnungen 14 auf. Gemäß einer bevorzugten Ausführungsform sind diese Maschenöffnungen 14 entlang der Mittelkettrichtung 3 ausgerichtet. Dies bedeutet, dass gegenüberliegende Seitenpaare von Längsstegen der rechteckigen Maschenöffnungen 14 parallel zu der Mittelkettrichtung 3 verlaufen. Gleichzeitig verlaufen gegenüberliegende Seitenpaare von Querstegen orthogonal hierzu und damit parallel zu einer Schussrichtung 15. Bei einer solchen Ausrichtung der einzelnen Maschen 14 können diese auch größere Zugkräfte aufnehmen ohne sich wesentlich zu verformen oder einzuschnüren. Auf diese Weise bleibt die effektive Porosität des Mittelabschnitts 2 gewahrt und es wird sichergestellt, dass bereits entstandene Einwachsungen des Mittelabschnitts 2 mit dem umgebenden Gewebe nicht wieder aufgerissen werden. Die Folge ist eine schneller entstehende und dauerhafte bleibende Verbindung der bandartigen Struktur 1 mit dem Gewebe.

Besondere Vorteile ergeben sich, wenn die rechteckigen Maschenöffnungen 14 in Mittelkettrichtung 3 länger sind als in einer Schussrichtung 15 des Mittelabschnitts 2. Schwerpunktmäßig wird die bandartige Struktur 1 in ihrer Hauptrichtung auf Zug beansprucht. Dadurch, dass die in Mittelkettrichtung 3 verlaufenden Kettstäbchen aus meist mehreren, durch Fransen gebildeten Kettfäden zusammengesetzt sind, ist das Mittelgewirke 2a in dieser Richtung besonders zugfest. Aus diesem Grund kann auch der Abstand der die rechteckigen Maschenöffnungen 14 bildenden Querstege in Mittelkettrichtung 3 größer gewählt werden als der Abstand der Längsstege in Schussrichtung 15 ohne die Zugfestigkeit in der Hauptbelastungsrichtung zu beeinträchtigen. Dafür vergrößert sich hierdurch die Fläche der Maschenöffnungen 14, was zu einer verbesserten Porosität und insbesondere zu einer verbesserten effektiven Porosität führt.

Wie bereits beschrieben wurde, wird die bandartige Struktur 1 jeweils durch eine Öffnung in das Peritoneum hinein- und wieder hinausgeführt. Um dies zu erleichtern ist bevorzugt vorgesehen, dass die bandartige Struktur 1 eine rohrartige Einführhilfe 16 umfasst, welche an dem Seitenabschnitt 4 befestigt ist. Die rohrartige Form erleichtert den Durchtritt durch die Öffnung in das Peritoneum. Die rohrartige Einführhilfe 16 kann beispielsweise in der Art eines Schrumpfschlauches ein Ende des Seitenabschnitts 4 einfassen und auf diese Weise mit dem Seitenabschnitt 4 verbunden sein. Die rohrartige Einführhilfe 16 kann ihrerseits auch mit einem Zugfaden 17 verbunden sein, an welchem die rohrartige Einführhilfe 16 und mit ihr die ganze bandartige Struktur 1 gezogen werden kann. Sowohl die Einführhilfe 16 als auch der Zugfaden 17 werden dann durch das Zuschneiden der bandartigen Struktur 1 am Seitenabschnitt 4 abgetrennt und können dann entfernt werden. Bevorzugt ist ebenso, dass die Einführhilfe 16 mit der Mehrzahl von Fäden 5a, 5b, 5c verbunden ist. Auf diese Weise wird die auf die Einführhilfe 16 ausgeübte Zugkraft unmittelbar auf die für die Aufnahme der Zugkraft ausgelegten Bestandteile der bandartigen Struktur 1 übertragen.

Bevorzugt bestehen der Mittelabschnitt 2, der Seitenabschnitt 4 und die Mehrzahl von Fäden 5a, 5b, 5c der bandartigen Struktur 1 aus nicht resorbierbarem, biostabilem Polyvinylidenfluorid (PVDF). Weiter bevorzugt ist, dass dies auch für den Verbindungsabschnitt 6 gilt.

Weiter vorzugsweise umfasst die bandartige Struktur 1 mindestens einen bei einer Magnetresonanztomographie sichtbaren Zusatzstoff. Grundsätzlich ist das für die Herstellung verwendete Material von bandartigen Strukturen, welche als Implantat dienen sollen, nicht in einer Magnetresonanztomographie zu erkennen. Das gilt auch für PVDF. Durch den regelmäßigen Aufbau der vorschlagsgemäßen bandartigen Struktur 1 reicht schon der Zusatz von bei einer Magnetresonanztomographie sichtbaren Stoffen zu nur wenigen Fäden, um die derzeitige Anordnung der bandartigen Struktur im Körper erkennbar zu machen. Auf diese Weise kann der weitere Verlauf der Therapie beobachtet werden, ohne dass chirurgisch eingegriffen werden müsste.

## Patentansprüche

1. Bandartige Struktur (1) zur Augmentation eines Ligaments, umfassend
- einen Mittelabschnitt (2) zur Befestigung an einem Organ des kleinen Beckens,
- einen Seitenabschnitt (4) zur Befestigung an einer Aufhängungsfläche und
- eine Mehrzahl von Fäden (5a, 5b, 5c), welche mit dem Mittelabschnitt (2) und dem Seitenabschnitt (4) verbunden sind, wobei die bandartige Struktur (1)
- einen Verbindungsabschnitt (6) aufweist,
welcher Verbindungsabschnitt (6) die Mehrzahl von Fäden (5a, 5b, 5c) umfasst, wobei der Verbindungsabschnitt (6) zwischen dem Mittelabschnitt (2) und dem Seitenabschnitt (4) so angeordnet ist, dass eine auf den Mittelabschnitt (2) und den Seitenabschnitt (4) ausgeübte Zugkraft zumindest abschnittsweise vollständig von dem Verbindungsabschnitt (6), vorzugsweise von der Mehrzahl von Fäden (5a, 5b, 5c), übertragen wird und wobei der Verbindungsabschnitt (6, 7) einen Verzwirbelungsabschnitt (9) aufweist, welcher aus der Mehrzahl von Fäden (5a, 5b, 5c) besteht,
**dadurch gekennzeichnet, dass**
der Mittelabschnitt (2) ein Mittelgewirke (2a) mit einer Mittelkettrichtung (3) aufweist, dass das Mittelgewirke (2a) die Mehrzahl von Fäden (5a, 5b, 5c) umfasst und dass die Mehrzahl von Fäden (5a, 5b, 5c) in dem Verzwirbelungsabschnitt (9) getrennt geführt ist.

2. Bandartige Struktur (1) nach Anspruch 1, **dadurch gekennzeichnet, dass** die bandartige Struktur (1)
- einen zweiten Verbindungsabschnitt (7) und
- einen zweiten Seitenabschnitt (8) aufweist,
wobei je ein Verbindungsabschnitt (6, 7) zwischen dem Mittelabschnitt (2) und den Seitenabschnitten (4, 8) so angeordnet ist, dass eine auf die beiden Seitenabschnitte (4, 8) ausgeübte Zugkraft zumindest abschnittsweise vollständig von den Verbindungsabschnitten (6, 7), vorzugsweise von der Mehrzahl von Fäden (5a, 5b, 5c), übertragen wird.

3. Bandartige Struktur (1) nach Anspruch 1 oder 2, **dadurch gekennzeichnet, dass** die Mehrzahl von Fäden (5a, 5b, 5c) abschnittsweise ein Textilband (10) bildet, in welchem die Mehrzahl von Fäden (5a, 5b, 5c) durch Bindefasern (11) miteinander verbunden ist.

4. Bandartige Struktur (1) nach einem der Ansprüche 1 bis 3, **dadurch gekennzeichnet, dass** die Mehrzahl von Fäden (5a, 5b, 5c) Kettfäden (12) in dem Mittelgewirke (2a) bildet.

5. Bandartige Struktur nach einem der Ansprüche 1 bis 4, **dadurch gekennzeichnet, dass** der Seitenabschnitt (4, 8)
- ein Seitengewirke (4a, 8a) aufweist, vorzugsweise, aus dem Seitengewirke (4a, 8a) besteht, welches Seitengewirke (4a, 8a) die Mehrzahl von Fäden (5a, 5b, 5c) umfasst,
vorzugsweise, dass die Mehrzahl von Fäden (5a, 5b, 5c) Kettfäden (12) in dem Seitengewirke (4a, 8a) bildet, insbesondere, dass das Seitengewirke (4a, 8a) die Bindefasern umfasst.

6. Bandartige Struktur nach Anspruch 5, **dadurch gekennzeichnet, dass** die Mehrzahl von Fäden (5a, 5b, 5c) so angeordnet ist, dass sie, vorzugsweise regelmäßig beabstandete, Markierungen (13) zur Kennzeichnung von Schneideoder Verbindungsstellen bildet.

7. Bandartige Struktur (1) nach einem der Ansprüche 1 bis 6, **dadurch gekennzeichnet, dass** die bandartige Struktur (1)
- eine gemeinsame Kettrichtung (3a) aufweist,
vorzugsweise, dass die Mehrzahl von Fäden (5a, 5b, 5c) entlang der bandartigen Struktur (1) Kettfäden bilden,
insbesondere, dass alle Kanten, der bandartigen Struktur (1) entlang der gemeinsamen Kettrichtung (3a) echte Kanten sind.

8. Bandartige Struktur (1) nach einem der Ansprüche 1 bis 7, **dadurch gekennzeichnet, dass** der Mittelabschnitt (2)
- rechteckige Maschenöffnungen (14) aufweist,
vorzugsweise, dass die rechteckigen Maschenöffnungen (14) entlang der Mittelkettrichtung (3) ausgerichtet sind, insbesondere, dass die rechteckigen Maschenöffnungen (14) in Mittelkettrichtung (3) länger sind als in einer Schussrichtung (15) des Mittelabschnitts (2).

9. Bandartige Struktur (1) nach einem der Ansprüche 1 bis 8, **dadurch gekennzeichnet, dass** der Mittelabschnitt (2), der Seitenabschnitt (4) und die Mehrzahl von Fäden (5a, 5b, 5c), insbesondere auch der Verbindungsabschnitt (6) aus nicht resorbierbarem, biostabilem Polyvinylidenfluorid bestehen.

10. Bandartige Struktur (1) nach einem der Ansprüche 1 bis 9, **dadurch gekennzeichnet, dass** die bandartige Struktur (1)
- eine rohrartige Einführhilfe (16) umfasst, welche an dem Seitenabschnitt (4) befestigt ist,
vorzugsweise, dass die Einführhilfe (16) mit der Mehrzahl von Fäden (5a, 5b, 5c) verbunden ist.

11. Bandartige Struktur nach einem der Ansprüche 1 bis 10, **dadurch gekennzeichnet, dass** sie
- mindestens einen bei einer Magnetresonanztomographie sichtbaren Zusatzstoff umfasst.

12. Verfahren zur Herstellung einer bandartigen Struktur nach einem der Ansprüche 1 bis 11 mit einer Häkelgalonmaschine.

## Claims

1. Band-like structure (1) for the augmentation of a ligament, comprising
- a central section (2) for fastening to an organ of the true pelvis,
- a lateral section (4) for fastening to a suspension surface, and
a plurality of threads (5a, 5b, 5c) which are connected to the central section (2) and the lateral section (4),
wherein the band-like structure (1)
- has a connecting section (6),
- said connecting section (6) comprising the plurality of threads (5a, 5b, 5c), wherein the connecting section (6) is located between the central section (2) and the lateral section (4) such that a tensile force exerted on the central section (2) and the lateral section (4) is at least sectionally completely transmitted by the connecting section (6), preferably by the plurality of threads (5a, 5b, 5c), and wherein the connecting section (6, 1 7) has a contortion section (9) which consists of the plurality of threads (5a, 5b, 5c),
**characterized in that**
the central section (2) has a central warp-knitted fabric (2a) with a central warp direction (3), **in that** the central warp-knitted fabric (2a) comprises the plurality of threads (5a, 5b, 5c) and **in that** the plurality of threads (5a, 5b, 5c) is separately guided within the contortion section (9).

2. Band-like structure (1) according to Claim 1, **characterized in that** the band-like structure (1) has
- a second connecting section (7) and
- a second lateral section (8),
wherein a connecting section (6, 7) is respectively located between the central section (2) and the lateral sections (4, 8) such that a tensile force exerted on the two lateral sections (4, 8) is at least sectionally completely transmitted by the connecting sections (6, 7), preferably by the plurality of threads (5a, 5b, 5c).

3. Band-like structure (1) according to Claim 1 or 2, **characterized in that** the plurality of threads (5a, 5b, 5c) sectionally forms a textile band (10) in which the plurality of threads (5a, 5b, 5c) is interconnected by binding fibres (11).

4. Band-like structure (1) according to one of Claims 1 to 3, **characterized in that** the plurality of threads (5a, 5b, 5c) forms warp threads (12) in the central warp-knitted fabric (2a).

5. Band-like structure according to one of Claims 1 to 4, **characterized in that** the lateral section (4, 8)
- has a lateral warp-knitted fabric (4a, 8a), preferably consists of the lateral warp-knitted fabric (4a, 8a), said lateral warp-knitted fabric (4a, 8a) comprising the plurality of threads (5a, 5b, 5c),
preferably that the plurality of threads (5a, 5b, 5c) forms warp threads (12) in the lateral warp-knitted fabric (4a, 8a), in particular that the lateral warp-knitted fabric (4a, 8a) comprises the binding fibres.

6. Band-like structure according to Claim 5, **characterized in that** the plurality of threads (5a, 5b, 5c) is arranged such that it forms markings (13), which are preferably spaced in a regular manner, for identifying cutting or connecting locations.

7. Band-like structure (1) according to one of Claims 1 to 6, **characterized in that** the band-like structure (1)
- has a common warp direction (3a),
preferably that the plurality of threads (5a, 5b, 5c) form warp threads along the band-like structure (1),
in particular, that all selvedges of the band-like structure (1) along the common warp direction (3a) are genuine selvedges.

8. Band-like structure (1) according to one of Claims 1 to 7, **characterized in that** the central section (2)
- has rectangular mesh openings (14),
preferably that the rectangular mesh openings (14) are oriented along the central warp direction (3), in particular that the rectangular mesh openings (14) are longer in the central warp direction (3) than in a weft direction (15) of the central section (2).

9. Band-like structure (1) according to one of Claims 1 to 8, **characterized in that** the central section (2), the lateral section (4) and the plurality of threads (5a, 5b, 5c), in particular also the connecting section (6), consist of non-absorbable, bio-stable polyvinylidene fluoride.

10. Band-like structure (1) according to one of Claims 1 to 9, **characterized in that** the band-like structure (1)
- comprises a tubular insertion aid (16), which is fastened to the lateral section (4),
preferably that the insertion aid (16) is connected to the plurality of threads (5a, 5b, 5c).

11. Band-like structure according to one of Claims 1 to 10, **characterized in that** it
- comprises at least one additive which is visible in magnetic resonance tomography.

12. Method for the manufacture of a band-like structure according to one of Claims 1 to 11, using a crochet galloon machine.

## Revendications

1. Structure (1) de type en ruban servant à favoriser la croissance d'un ligament et comprenant :
une section centrale (2) destinée à être fixée à un organe du petit bassin,
une section latérale (4) destinée à être fixée sur une surface de suspension et
plusieurs fils (5a, 5b, 5c) reliés à la section centrale (2) et à la section latérale (4), la structure (1) de type en ruban présentant une section de liaison (6),
la section de liaison (6) comportant les différents fils (5a, 5b, 5c),
la section de liaison (6) étant disposée entre la section centrale (2) et la section latérale (4) de telle sorte qu'une force de traction exercée sur la section centrale (2) et sur la section latérale (4) soit transférée complètement au moins dans certaines parties par la section de liaison (6) de préférence par les différents fils (5a, 5b, 5c), la section de liaison (6, 7) présentant une section de torsadage (9) constituée des différents fils (5a, 5b, 5c), **caractérisée en ce que**
la section centrale (2) présente un tricot central (2a) présentant une direction (3) de chaîne centrale,
**en ce que** le tricot central (2a) comporte les différents fils (5a, 5b, 5c) et
**en ce que** les différents fils (5a, 5b, 5c) sont guidés séparément dans la section de torsadage (9).

2. Structure (1) de type en ruban selon la revendication 1, **caractérisée en ce que** la structure (1) de type en ruban présente une deuxième section de liaison (7) et une deuxième section latérale (8), une section de liaison (6, 7) étant disposée entre la section centrale (2) et chacune des sections latérales (4, 8) de telle sorte qu'une force de traction exercée sur les deux sections latérales (4, 8) soit transférée complètement au moins par partie par les sections de liaison (6, 7) et de préférence par les différents fils (5a, 5b, 5c).

3. Structure (1) de type en ruban selon les revendications 1 ou 2, **caractérisée en ce que** les différents fils (5a, 5b, 5c) forment par partie un ruban textile (10) dans lequel les différents fils (5a, 5b, 5c) sont reliés les uns aux autres par des fils de liaison (11).

4. Structure (1) de type en ruban selon l'une des revendications 1 à 3, **caractérisée en ce que** les différents fils (5a, 5b, 5c) forment des fils de chaîne (12) du tricot central (2a).

5. Structure de type en ruban 1 à 4, **caractérisée en ce que** la section latérale (4, 8) présente un tricot latéral (4a, 8a), et de préférence est constituée du tricot latéral (4a, 8a), le tricot latéral (4a, 8a) comportant les différents fils (5a, 5b, 5c) et de préférence **en ce que** les différents fils (5a, 5b, 5c) forment des fils de chaîne (12) du tricot latéral (4a, 8a) et en particulier que le tricot latéral (4a, 8a) comporte les fibres de liaison.

6. Structure de type en ruban selon la revendication 5, **caractérisée en ce que** les différents fils (5a, 5b, 5c) sont disposés de telle sorte qu'ils forment des repères (13) de caractérisation d'emplacement de coupe ou de liaison, situés de préférence à intervalles réguliers.

7. Structure (1) de type en ruban selon l'une des revendications 1 à 6, **caractérisée en ce que** la structure (1) de type en ruban présente une direction commune de chaîne (3a), de préférence **en ce que** les différents fils (5a, 5b, 5c) forment des fils de chaîne le long de la structure (1) en forme de ruban et en particulier **en ce que** toutes les lisières de la structure (1) en ruban sont des lisières véritables le long de la direction commune de chaîne (3a).

8. Structure (1) de type en ruban selon l'une des revendications 1 à 7, **caractérisée en ce que** la section centrale (2) présente des ouvertures rectangulaires (14) de maille, de préférence **en ce que** les ouvertures rectangulaires (14) de maille sont orientées le long de la direction (3) de la chaîne centrale et en particulier **en ce que** les ouvertures rectangulaires (14) de mailles sont plus longues dans la direction (3) de chaîne centrale que dans une direction de trame (15) de la section centrale (2).

9. Structure (1) de type en ruban selon l'une des revendications 1 à 8, **caractérisée en ce que** la section centrale (2), la section latérale (4), les différents fils (5a, 5b, 5c) et en particulier aussi la section de liaison (6) sont constitués de poly(fluorure de vinylidène) biostable et non résorbable.

10. Structure (1) de type en ruban selon l'une des revendications 1 à 9, **caractérisée en ce que** la structure (1) en forme de ruban comporte un accessoire tubulaire d'insertion (16) fixé sur la section latérale (4) et de préférence **en ce que** l'accessoire d'insertion (16) est relié aux différents fils (5a, 5b, 5c).

11. Structure de type en ruban selon l'une des revendications 1 à 10, **caractérisée en ce qu'**elle comporte au moins un matériau d'addition visible en tomographie à résonance magnétique.

12. Procédé de fabrication d'une structure de type en ruban selon l'une des revendications 1 à 11 à l'aide d'une machine à crocheter.
